# EUROPEAN PATENT SPECIFICATION

(11) **EP 3 551 295 B1**
(45) Date of publication and mention of the grant of the patent: **02.02.2022**
(21) Application number: 17808507.2
(22) Date of filing: 06.12.2017
(51) Int. Cl.: A61Q 1/00, A61Q 3/00, A61Q 5/00, A61K 8/18, B41M 3/00, B41J 3/407, A61K 8/25, A61K 8/26, A61K 8/29, A61K 8/60, A61K 8/19, B44C 1/165

(54) **PROCESS FOR PRODUCING A COSMETIC ARTICLE**
VERFAHREN ZUR HERSTELLUNG EINES KOSMETISCHEN ARTIKELS
PROCÉDÉ DE PRODUCTION D'UN ARTICLE COSMÉTIQUE

(30) Priority: 09.12.2016 FR 1662230
(43) Date of publication of application: 16.10.2019
(73) Proprietor: L'Oréal, 75008 Paris (FR)
(72) Inventor: GIRON, Franck, 94152 Chevilly Larue (FR); SAMAIN, Henri, 94152 Chevilly Larue (FR)
(74) Representative: Nony
(86) International application number: PCT/EP2017/081644
(87) International publication number: WO 2018/104374

(56) References cited:
- WO-A1-2015/097613
- WO-A1-2015/097619
- TechCrunch: "Print Your Own Makeup With Mink | Disrupt NY 2014", , 5 May 2014 (2014-05-05), page 1 pp., XP054976603, Retrieved from the Internet: URL:https://www.youtube.com/watch?v=cBZHFU QiP8Q [retrieved on 2016-06-17]

## Description

### Field of the invention

The present invention relates to the cosmetic field, especially to making up human keratin materials, in particular the skin, lips, nails or keratin fibers such as the hair, eyelashes or eyebrows.

The invention relates in particular but not exclusively to the production of a cosmetic article and to the application of makeup using this article, by removal or by transfer.

### Background

There is an interest in being able to apply personalized makeup. The personalization may relate to the color of the makeup composition, that the user seeks for example to match exactly to that of the skin, or to a pattern that the user wishes to transfer to the skin.

It has been envisaged to deposit a product directly onto the skin using an inkjet printer head, with the aid of a suitable device.

FR 3 015 888 relates to a process for making up an area of human keratin materials using a makeup device. The device comprises a deformable substrate bearing a transfer surface intended to come into contact with the keratin materials and configured to receive a coat of cosmetic ink by means of at least one digital printer.

It has furthermore been envisaged to deposit a product in powder form, here also referred to as "toner", with the aid of a laser printer. It is then known to use a laser printer without a fuser or with a deactivatable fuser so as not to deteriorate the product, such as for example proposed in WO 2015/097619 or in FR 3 016 054.

The use of the powders is intended, in this case, to cover the human keratin materials. The problem of the bacteriological quality of these powders thus arises. This problem is even more significant since the powders may remain in their packaging for a long time, since the users may themselves produce their toner by introducing mixtures of powders into a suitable packaging, and since the users may wish to obtain cosmetic effects other than colored effects alone, these effects sometimes being obtained with the aid of bacteriologically unstable compounds.

The invention thus aims to propose a solution that makes it possible to produce a cosmetic article, in particular a cosmetic composition, and to apply this cosmetic composition to human keratin materials, while ensuring a bacteriological quality of this composition so that it can be used without risk for a cosmetic application.

The invention also aims to propose the use of a laser printer with heating unit to produce such a cosmetic article.

### Process for producing a cosmetic article

According to a first one of its aspects, the present invention relates to a process for producing a cosmetic article, in particular a cosmetic composition, by printing using a laser printer comprising a heating unit and at least one cosmetic toner, the process comprising the steps consisting in:
a) producing, with the printer, a printing on a support with the aid of the cosmetic toner,
b) heating the support coated with the printing with the aid of the heating unit to an operating temperature greater than or equal to 60°C, preferably greater than or equal to 80°C, better still greater than or equal to 120°C, the cosmetic toner comprising a weight content of material that is fusible at the operating temperature of less than 10%, better still less than 5%, relative to the total weight of the toner.

Owing to the invention, by heating the support coated with the printing and therefore the printing, it is possible to improve the bacteriological quality of the cosmetic article manufactured. Specifically, the heating exerts a biocidal activity that makes it possible for example to eliminate at least a portion of the germs of any type, in particular bacteria, from the cosmetic article.

The laser printer is preferably arranged to enable the formation, by electrophotography or magnetophotography, of an image that forms the printing on the support using the cosmetic toner.

The term "cosmetic toner" should be understood as meaning a pulverulent cosmetic composition that is compatible with the formation of an image via an electrophotographic or magnetophotographic process as used in conventional laser printers. Preferably, it is a toner that is suitable for electrophotographic use.

The toner is cosmetic in the sense that it is compatible with an application to human keratin materials, and in particular the skin. Depending on the surface to be made up, the formulation of the toner may be different. For example, for an application to the hair or the nails, it is possible to use certain compounds that might not be used for an application to certain areas of the body such as the lips, for example angular microfacetted pigments capable of injuring the mucous membranes.

In examples of the implementation of the process according to the invention, the printer is advantageously configured to deliver the toner present on the support in a sufficiently free state to enable the removal thereof or transfer thereof by contact with the human keratin materials.

The printer may be a color or monochrome laser printer.

The invention makes it possible to electrostatically or electromagnetically deposit a cosmetic toner on the support for example according to a precise pattern that can be personalized on demand, as a function of an image file sent to the printer.

The printer may be arranged in order to transfer the toner deposited on the drum of the printer to the support, consisting of a substrate in sheet form and/or a transfer roller. In this case, the transfer roller may be rotated when the latent image is transferred.

The heating step b) advantageously takes place at the same time as the printing step a) or after the printing step a). In the case where the heating step b) takes place after the printing step a), the implementation of step b) takes place preferably less than 10 s, in particular less than 1 s, after the end of step a).

In the case where the heating step b) takes place at the same time as step a), the heating may be carried out via the back of the support. In the case where the heating step b) takes place after step a), the heating may be carried out via the front of the support and/or via the back of the support.

The heating unit, which may also be referred to as a fuser or fixing unit, may have a working temperature greater than or equal to 180°C, better still greater than or equal to 200°C.

The "working temperature" is understood as meaning the temperature of the heating element of the heating unit. The "operating temperature" is understood as meaning the temperature to which the toner is brought.

The heating unit may be chosen from the group formed by an element, in particular in the form of a toner fuser roller, a UV, visible or IR lamp, the emission spectrum being chosen in order to be absorbed by the toner or the support itself, and a combination thereof. The heating unit comprises for example a toner fuser roller, made of aluminum, which may be coated with a nonstick compound, for example PTFE, and in particular heated from the inside by a halogen lamp.

When the heating unit comprises a toner fuser roller, this roller may be capable of occupying a position spaced apart from the support, in particular by several millimeters, for example by 1 to 20 mm, so as to transmit a predetermined heat to the support coated with the printing. In this case, the process comprises for example the step consisting in placing the toner fuser roller in the spaced-apart position before the printing step a). The fuser roller may be capable furthermore of occupying a position close to the support, so as to press on the support, in particular for office use with a toner that may be non-cosmetic for example, which renders the printer multipurpose.

Preferably, the operating temperature of the printer is controllable, optionally by software means, to produce printing on the support with a cosmetic toner in order to carry out the disinfection without giving rise to fusion of the optional fusible compound(s) of the toner.

The printer may optionally be configured to enable the deactivation, optionally by software means, of the heating unit. This may make it possible to use the printer in another use comprising printing on the support with a toner comprising one or more particularly temperature-sensitive compounds, for example agents with a biological effect such as vitamins, and/or proteins.

As indicated above, the printer may advantageously be produced so as to be able to be used for uses other than the production of a cosmetic article, in particular for office use. It is then possible to use the printer with a toner containing waxes and/or fusible polymers in an amount greater than 10% relative to the total weight of the toner, without implementing the process according to the invention.

The printer may be configured to automatically recognize the presence of a cosmetic toner, or even to identify this cosmetic toner, and to heat the heating unit to a predetermined temperature depending on the nature of the toner thus identified.

### Cosmetic toner

The toner used in the process according to the invention is suitable for a cosmetic application. It is thus non-toxic with respect to the region to which it is applied.

The toner may already be used for conventional laser printing, if the formulation of the toner is suitable for use in cosmetics.

The cosmetic toner may be contained in a toner cartridge forming a reservoir. Preferably, this cartridge is removable, in order to allow the replacement thereof. The cartridge may belong to an assembly comprising a mechanism for metering and homogenizing the toner before the transfer thereof to the drum, and optionally also a reservoir for recovering the toner present in excess on the drum after formation of the latent image.

Preferably, the printer uses several cosmetic toner cartridges, especially several cartridges of different colors, or one cartridge with multiple compartments enabling the deposition of multiple compositions on the support.

The use of several toners makes it possible, on the one hand, to print a pattern in the color that is desired, therefore to offer the user the possibility of coating the support with a composition having the color of their choice, with a view to then removing it or transferring it to the keratin materials. On the other hand, the user may produce complex and precise images, of high resolution, in order to transfer them to the keratin materials.

When the laser printer comprises a plurality of different cosmetic toners, the process may consist in carrying out steps a) and b) several times with the various toners, so as to produce several different printings on the support.

The choice of the known toners that can be used may be wider for certain applications, for example to the hair, eyelashes or nails, than for others, for example to the skin or lips.

The cosmetic toner may not comprise any material that is fusible at the operating temperature. Thus, according to the invention, the cosmetic toner used in the process according to the invention preferably contains no or contains little, in particular less than 10% by weight or even less than 5% by weight, material that is fusible at the operating temperature.

When present, the fusible material is for example chosen from waxes, fusible polymers or polymers that can be softened at a temperature below 120°C, and mixtures thereof.

The expression "fusible polymer or polymer that can be softened" is understood as meaning any polymer capable of exhibiting, by heating beyond a given temperature, a change of state from the solid state to a state that ranges from rubbery to liquid (melting). In general, melting only occurs for semicrystalline polymers (only the crystalline portion is affected). Non-crystalline polymers may soften (without becoming liquid) when they are subjected to a temperature above their glass transition temperature (Tg), and in particular when they are subjected to a temperature at least 10°C above their glass transition temperature.

When the fusible material is a wax, it may in particular be chosen from waxes of animal, plant, mineral or synthetic origin, in particular chosen from ester waxes, in particular fatty acid ester waxes and preferably polyol-fatty acid ester waxes, alcohol waxes, paraffin waxes, and silicone waxes, preferably an alcohol wax, and more preferentially cetyl alcohol and/or stearyl alcohol.

*"Ester wax"* is understood to mean, according to the invention, a wax comprising at least one ester function. Use may especially be made, as ester waxes, of a wax chosen from the waxes of formula R₁COOR₂ in which R₁ and R₂ represent linear, branched or cyclic aliphatic chains in which the number of atoms ranges from 10 to 50, which may contain a heteroatom such as O, N or P, di(1,1,1-trimethylolpropane) tetrastearate, sold under the name Hest 2T-4S^{®} by the company Heterene, diester waxes of a dicarboxylic acid of general formula R³-(-OCO-R⁴-COO-R⁵) in which R³ and R⁵ are identical or different, preferably identical, and represent a C₄-C₃₀ alkyl group (alkyl group comprising from 4 to 30 carbon atoms) and R⁴ represents a linear or branched C₄-C₃₀ aliphatic group (alkyl group comprising from 4 to 30 carbon atoms) which may or may not contain one or more unsaturations, and is preferably linear and unsaturated.

According to another embodiment, the wax may be an alcohol wax. "*Alcohol wax*" is understood to mean, according to the invention, a wax comprising at least one alcohol function, that is to say comprising at least one free hydroxyl (OH) group. Examples of alcohol waxes that may be mentioned include the C₃₀-₅₀ alcohol wax Performacol^{®} 550 Alcohol sold by the company New Phase Technologies, stearyl alcohol and cetyl alcohol.

"*Silicone wax*" is understood to mean an oil comprising at least one silicon atom and especially comprising Si-O groups. Among the commercial silicone waxes of this type, mention may be made in particular of those sold under the names Abilwax 9800, 9801 or 9810 (Goldschmidt), KF910 and KF7002 (Shin-Etsu), or 176-1118-3 and 176-11481 (General Electric). The silicone waxes that may be used may also be substituted polysiloxanes, alkyl or alkoxy dimethicones, and also (C₂₀-C₆₀)alkyl dimethicones, in particular (C₃₀-C₄₅)alkyl dimethicones, such as the silicone wax sold under the name SF-1642 by the company GE-Bayer Silicones or C₃₀-₄₅ alkyl dimethylsilyl polypropylsilsesquioxane under the name SW-8005^{®} C30 Resin Wax sold by the company Dow Corning.

The fusible polymers are preferably thermoplastic polymers and/or semicrystalline polymers.

For the purposes of the present invention, the term "*thermoplastic polymer*" means a polymer which softens when hot and which can be molded while retaining its shape after cooling. The thermoplastic polymers that can be used in the context of the present invention are any polymer or copolymer or any blend of polymers and/or copolymers having the property of being thermoplastic. Among the thermoplastic polymers, mention may be made especially of polyolefins, such as polypropylene, polyethylene or ethylenic copolymers, especially Surlyn^{®}, polystyrenes such as polystyrene, acrylonitrile butadiene styrene (ABS) and styrene acrylonitrile copolymer (SAN), polyamides, polyacrylates such as polyoxymethylene, polyvinyl chloride, polyethylene terephthalate, and mixtures thereof. Mention may also be made of aliphatic polyesters, and in particular polyethylene terephthalate (PET), polybutylene terephthalate (PBT), polyhydroxyalkanoates (PHAs), such as poly-3-hydroxybutyrate (PHB), polyhydroxyvalerate (PHV) or polyhydroxyhexanoate (PHH), polylactic acids (PLAs), polybutylene succinates (PBSs), polycaprolactones (PCLs), polyanhydrides, polyvinyl alcohols, and derivatives thereof, acetate esters, such as acetate/polyvinyl (PVAc) copolymer, starch derivatives, polysaccharides, including in particular cellulose derivatives such as cellulose esters, and derivatives thereof, in particular celluloids or cellulose ethers, and mixtures thereof. In particular, among the cellulose esters, mention may be made of cellulose acetate, cellulose triacetate, cellulose propionate, cellulose acetate propionate, cellulose acetate butyrate, and cellulose sulfate, and mixtures thereof. Among the cellulose ethers, mention may in particular be made of methyl cellulose, ethyl cellulose, ethyl methyl cellulose, hydroxyethyl cellulose, hydroxypropyl cellulose (HPC), hydroxyethyl methyl cellulose, hydroxypropyl methyl cellulose (HPMC), ethyl hydroxyethyl cellulose, carboxymethyl cellulose (CMC), and mixtures thereof. Among the acetate esters, mention may in particular be made of acetate/polyvinyl copolymers, including in particular ethylene-vinyl acetate (EVA) and derivatives thereof. For example, mention may be made of EVA/ethyl cellulose or EVA/starch copolymers.

For the purposes of the invention, the term "*semicrystalline polymer*" means polymers comprising a crystallizable portion and an amorphous portion and having a first-order reversible change of phase temperature, in particular melting (solid-liquid transition) temperature.

The semicrystalline polymers of the invention may be of synthetic origin. In particular, the semicrystalline polymer may be chosen from homopolymers and copolymers comprising units resulting from the polymerization of one or more monomers bearing crystallizable hydrophobic side chain(s), polymers bearing in the backbone at least one crystallizable block, polycondensates of aliphatic or aromatic or aliphatic/aromatic polyester type, copolymers of ethylene and of propylene prepared by metallocene catalysis, and acrylate/silicone copolymers.

The semicrystalline polymers that may be used in the invention may be chosen in particular from block copolymers of polyolefins with controlled crystallization, the monomers of which are described in EP 0 951 897, polycondensates especially of aliphatic or aromatic or aliphatic/aromatic polyester type, copolymers of ethylene and of propylene prepared by metallocene catalysis, homopolymers or copolymers bearing at least one crystallizable side chain and homopolymers or copolymers bearing in the backbone at least one crystallizable block, such as those described in patent US 5 156 911, homopolymers or copolymers bearing at least one crystallizable side chain in particular bearing fluoro group(s), as described in document WO 01/19333, acrylate/silicone copolymers, and mixtures thereof.

The cosmetic toner may comprise only compounds that have a melting temperature and/or glass transition temperature Tg above 120°C, included in particular in the group formed by polycarbonates (around 150°C), polyvinylcaprolactams (typically 180°C), polyetheretherketones (in general 140°C), polyetherketones (in general 160°C), polyaryletherketones (in general 160°C), polyterephthalates (in general 170°C), polysulfones (in general 190°C) and polyetherimides (in general 210°C).

For the purposes of the invention, the melting temperature corresponds to the temperature of the most endothermic peak observed on thermal analysis (DSC) as described in standard ISO 11357-3; 1999. The melting point may be measured using a differential scanning calorimeter (DSC), for example the calorimeter sold under the name *MDSC 2920* by the company TA Instruments or else the calorimeter sold under the name *DSC 30* by the company METLER.

The measurement protocol is as follows:
The sample of 5 mg placed in a crucible is subjected to a first temperature rise ranging from -20°C to 100°C, at the heating rate of 10°C/minute, is then cooled from 100°C to -20°C at a cooling rate of 10°C/minute and is finally subjected to a second temperature rise ranging from -20°C to 100°C at a heating rate of 5°C/minute. During the second temperature rise, the variation of the difference in power absorbed by the empty crucible and by the crucible containing the sample is measured as a function of the temperature. The melting point of the sample is the temperature value corresponding to the top of the peak of the curve representing the variation of the difference in power absorbed as a function of the temperature.

The cosmetic toner may form a pulverulent composition comprising colored particles having a size suitable for use within a toner, and that are compatible with a cosmetic application.

The cosmetic toner may comprise at least one compound chosen from the group formed by at least one colorant, opacifying particles, anticaking particles, transparent particles, scattering white particles, and a mixture thereof. The opacifying or anticaking or transparent particles optionally present in the cosmetic toner are advantageously such that their melting temperature is above 120°C.

The toner may not have an antibacterial agent.

The toner according to the invention preferably comprises a colorant, which may be by itself or mixed with other powders.

The colorant(s) of the cosmetic toner is (are) advantageously not fusible at the operating temperature.

The colorant may be chosen from the dyes and pigments conventionally used in cosmetics, subject to the compatibility thereof with the printing process used within the printer.

In particular, the colorants may be chosen from pigments, nacres and reflective particles, and mixtures thereof, and preferably from pigments, in particular mineral pigments and lakes.

The pigments may be white or colored, mineral and/or organic, and coated or uncoated. Among the mineral pigments, mention may be made of metal oxides, in particular titanium dioxide, optionally surface-treated, zirconium, zinc or cerium oxides, and also iron, titanium or chromium oxides, manganese violet, ultramarine blue, chromium hydrate and ferric blue. Among the organic pigments that may be mentioned are carbon black, pigments of D & C type and lakes based on cochineal carmine or on barium, strontium, calcium or aluminum.

The nacres may be chosen from white nacreous pigments such as mica coated with titanium or with bismuth oxychloride, colored nacreous pigments such as titanium mica with iron oxides, titanium mica with in particular ferric blue or chromium oxide, titanium mica with an organic pigment of the abovementioned type, and also nacreous pigments based on bismuth oxychloride. Examples of nacres that may also be mentioned include natural mica coated with titanium oxide, with iron oxide, with natural pigment or with bismuth oxychloride. The nacres may more particularly have a yellow, pink, red, bronze, orangey, brown, gold and/or coppery color or glint.

The term "reflective particles" denotes particles whose size, structure, especially the thickness of the layer(s) of which they are made and their physical and chemical nature, and surface state, allow them to reflect incident light. This reflection may, if appropriate, have an intensity sufficient to create, at the surface of the composition or mixture, when the latter is applied to the support to be made up, highlight points visible to the naked eye, that is to say more luminous points which contrast with their surroundings by appearing to sparkle. The reflective particles may be selected so as not to significantly alter the coloration effect generated by the colorants with which they are combined, and more particularly so as to optimize this effect in terms of color rendering. They may more particularly have a yellow, pink, red, bronze, orangey, brown, gold and/or coppery color or glint. These particles may have varied forms and may especially be in platelet or globular form, in particular in spherical form. Irrespective of their form, the reflective particles may or may not have a multilayer structure, and, in the case of a multilayer structure, may have, for example, at least one layer of uniform thickness, especially of a reflective material. When the reflective particles do not have a multilayer structure, they may be composed, for example, of metal oxides, especially titanium or iron oxides obtained synthetically.

When the reflective particles have a multilayer structure, they may comprise, for example, a natural or synthetic substrate, especially a synthetic substrate at least partially coated with at least one layer of a reflective material, especially of at least one metal or metallic material. The substrate may be made of one or more organic and/or mineral materials. More particularly, it may be chosen from glasses, ceramics, graphite, metal oxides, aluminas, silicas, silicates, especially aluminosilicates and borosilicates, and synthetic mica, and mixtures thereof, this list not being limiting. The reflective material may comprise a layer of metal or of a metallic material. Reflective particles are described especially in documents JP-A-09188830, JP-A-10158450, JP-A-10158541, JP-A-07258460 and JP-A-05017710.

Again as an example of reflective particles comprising a mineral substrate coated with a layer of metal, mention may also be made of particles comprising a silver-coated borosilicate substrate. Particles with a silver-coated glass substrate, in the form of platelets, are sold under the name Microglass Metashine REFSX 2025 PS by the company Toyal. Particles with a glass substrate coated with nickel/chromium/molybdenum alloy are sold under the name Crystal Star GF 550 and GF 2525 by this same company. Use may also be made of particles comprising a metal substrate, such as silver, aluminum, iron, chromium, nickel, molybdenum, gold, copper, zinc, tin, magnesium, steel, bronze or titanium, said substrate being coated with at least one layer of at least one metal oxide, such as titanium oxide, aluminum oxide, iron oxide, cerium oxide, chromium oxide, silicon oxides and mixtures thereof. Examples that may be mentioned include aluminum powder, bronze powder or copper powder coated with SiO₂ sold under the name Visionaire by the company Eckart.

Preferably, the pulverulent colorant(s) is (are) present in a content of between 1% and 90% by weight and preferably between 2% and 30% by weight relative to the total weight of the intermediate composition.

Preferably, the pulverulent compound(s) is (are) chosen from talc, perlite, mica, clay, kaolin, silica, titanium oxide, starch, and mixtures thereof.

The reflective particles may also be used as anticaking particles. Such particles are in particular chosen from silicas and silicates, in particular aluminosilicates. Preferably, the transparent particles are transparent polymers or transparent silicas.

Preferably, the scattering white particles are titanium oxides.

### Agent for controlling the charge

The toner may comprise an agent for controlling the charge, intended to promote the electrostatic transfer of the toner to the drum.

This agent may be chosen from those conventionally used, subject to the compatibility thereof with a cosmetic use.

Use may be made of those mentioned in application WO 2007/134171, namely quaternary ammonium salts, benzalkonium chloride, benzethonium chloride, cetrimide (trimethyltetradecylammonium bromide), cyclodextrins, silica, aluminum oxide, titanium dioxide, ferrite and carbon black.

### Particulate additive

The toner preferably comprises a particulate additive in order to improve the fluidity, the developability and the sensitivity to electrostatic charges thereof. The particles of the additive may have a mean size ranging from 5 nm to 2 µm, better still from 5 nm to 500 nm, and their specific surface area preferably ranges from 20 to 500 m²/g according to the BET method.

Such a particulate additive is in particular chosen from fillers.

The fillers may be chosen from those that are well known to a person skilled in the art and commonly used in cosmetic compositions. The fillers may be mineral or organic, and lamellar or spherical. Mention may be made of talc, mica, silica, kaolin, polyamide powders, for instance Nylon^{®} sold under the name Orgasol^{®} by the company Atochem, poly-β-alanine powders and polyethylene powders, powders of tetrafluoroethylene polymers, for instance Teflon^{®}, lauroyllysine, starch, boron nitride, expanded polymeric hollow microspheres such as those of polyvinylidene chloride/acrylonitrile, for instance those sold under the name Expancel^{®} by the company Nobel Industrie, acrylic powders such as those sold under the name Polytrap^{®} by the company Dow Corning, polymethyl methacrylate particles and silicone resin microbeads (for example Tospearls^{®} from Toshiba), precipitated calcium carbonate, magnesium carbonate and magnesium hydrogen carbonate, hydroxyapatite, hollow silica microspheres (Silica Beads^{®} from Maprecos), glass or ceramic microcapsules, metal soaps derived from organic carboxylic acids having from 8 to 22 carbon atoms and in particular from 12 to 18 carbon atoms, for example zinc, magnesium or lithium stearate, zinc laurate and magnesium myristate.

The filler is in particular chosen from particles of silica, alumina, titanium dioxide, zinc oxide, clay, mica, diatomaceous earths, red iron oxide, magnesium oxide, zirconium oxide, calcium carbonate, silicon carbide and silicon nitride.

Preferably, use is made of silica and/or titanium dioxide, preferably in combination, and preferably silica and/or titanium dioxide that have been rendered hydrophobic. The size of the silica and/or titanium dioxide particles is preferably less than 50 nm.

Preferably, the mixture of hydrophobic silica and hydrophobic titanium dioxide is in a proportion by weight of between 0.3% and 1.5% relative to the total weight of the toner.

The proportion of particulate additive, and in particular of filler, preferably ranges from 0.01% to 5% by weight and better still from 0.01% to 2% by weight relative to the total weight of the toner.

### Preparation of the toner

It is desirable for the toner particles to have a mean size D₅₀ between 1 and 16 µm, better still between 3 and 10 µm, with as narrow a distribution as possible about a mean value so as to improve the printing quality.

The toner particles may be prepared by any suitable method, for example involving milling and pulverizing, as disclosed in paragraphs [0075] to [0079] of US 2006/0093943 A1 or in the examples given in application WO 2007/134171.

### Magnetic carrier

The toner used in the process according to the invention may be used in combination with a magnetic carrier such as ferrite or iron particles, as disclosed in paragraphs [0082] and [0083] of US 2006/0093943 A1.

### Support

In one particular exemplary embodiment, especially in the case where the cosmetic article is intended to be applied by transfer to the human keratin materials, the support, or substrate, used for the printing, has at least one translucent or transparent area. The translucent or transparent area allows a user to see through the support and thus to visualize more easily the surface to be made up and/or treated before transferring the cosmetic toner. The presence of a translucent or transparent area thus advantageously contributes, in this case, toward facilitating the production of a precise makeup result on the keratin materials. The translucent or transparent area of the support may be totally or partly superposed with the printing that forms a layer of cosmetic toner, and especially may extend beyond it.

The layer of cosmetic toner may be superposed in its entirety on the translucent or transparent area of the support. As a variant, only part of the layer of cosmetic toner is superposed on the transparent area of the substrate.

The support may be made of a transparent or translucent material. In this case, the translucent or transparent area extends over the entire surface of the support.

As a variant, the support is opaque over all or part of its surface.

The support may comprise a material in sheet form, especially a transparent material. The support may be a flexible sheet or a rigid plate. It may be made of plastic (for example made of polyethylene or polystyrene). The support is preferentially based on a non-absorbent material, for example a plastic film. The support is advantageously nonporous, at least on the face intended to receive the printing.

The surface for receiving the printing, which may consist of a transfer surface of the support, may or may not be flat. The surface of the support for receiving the printing may be defined by all or part of the outer surface of an applicator roller, the surface of an applicator pad, an element in sheet form, a patch, the surface of a porous foam, especially a sponge, a wipe, a coarse brush, a fine brush or a flocked tip.

In the case of an applicator roller, this roller may have the form of a straight cylinder. In one variant, the roller has the form of an irregular cylinder, for example the form of an hourglass. In one variant, the roller is "premolded", i.e. it has an initial non-flat shape corresponding to the general shape of the area to be made up, for example of the lips, of an eye socket, of an ankle or of a forearm.

In one variant, the support is pressed at the time of transfer against an imprint of the area to be made up, so that the transfer surface reproduces the relief of the area to be made up. The transfer surface is defined, for example, by all or part of the surface of a deformable sheet mounted on the surface of an applicator roller or a pad.

The transfer surface may be elastically deformable. Thus, in a first configuration, the transfer surface may be flat, and, in a second configuration, the transfer surface may be curved.

In one variant, the support is configured so that the transfer surface takes a first form, for example substantially flat, during printing, and a second form, different from the first, during the application of the toner to the keratin materials. The second form advantageously corresponds to the form of the surface of the keratin materials intended to be coated with the toner, for example the form of the nails or of a part of the face.

The thickness of the support corresponds to its maximum dimension measured perpendicular to the transfer surface. The support may have a variable thickness, adapted to the area of the keratin materials to be made up.

In one exemplary embodiment, when the cosmetic toner printing produced on the support is intended to be applied to the cheeks and/or the nails, the support may have a thickness of greater than or equal to 1 mm, especially 3 mm, for example ranging from 1 to 5 mm.

In one exemplary embodiment, when the toner is intended to be applied to the area around the eyes and/or to the lips, the support may have a thickness of greater than or equal to 3 mm, especially 1 cm, for example ranging from 3 mm to 20 mm.

In one exemplary embodiment, when the toner is intended to be applied to the nose and/or in the area of the ears, the support may have a thickness of greater than or equal to 1 cm, especially 3 cm, for example ranging from 1 to 4 cm.

The support may be premolded.

In one exemplary embodiment, the support comprises a printed instruction. The instruction states, for example, the nature of the keratin materials intended to be made up with the toner or illustrates to scale, enlarged, reduced or otherwise and "right-side up" the pattern deposited "wrong-side up" on the support.

In one exemplary embodiment, the transfer surface is detachable from a part of the support.

The support may be reusable. For example, printing is performed on the support, which is accessible for the transfer or the removal, but does not leave the printer. Thus, after use, the printer can reintegrate the support, clean it and make it ready for a fresh printing. When the support on which the composition is deposited is a roller, the latter may then be removed from the printer in order to apply the composition to the keratin materials by transfer, by making the roller roll in contact with the skin for example. This roller may be firmly attached to a gripping portion that facilitates the handling thereof.

### Other steps of the process

The process may also comprise a step that consists in choosing the pattern to be printed, for example as a function of the region to be treated. The pattern to be printed may comprise a color gradation. As a variant, the pattern to be printed is in the form of a flat tint. The pattern to be printed may reproduce any pattern of use for makeup, for example a pattern that imitates a skin texture, to be applied by transfer. The process may comprise a step that consists in selecting a printing color from a palette displayed on a screen.

The process may also comprise the acquisition of an image of an area of the human keratin materials to be made up prior to the preparation of the support, and a step that consists in producing the printing in the form of a pattern as a function of the image thus acquired. This makes it possible, for example, to adapt the contour of the pattern to the relief and/or to the color of the area to be made up.

The printing may follow geometric rectification rules. In so far as the transfer surface is deformable during the application, the pattern will be geometrically deformed (for example extension in one of the two dimensions). As a result, the pattern is printed with a geometrical deformation (in the present case, reduction along the deformable dimension(s)) such that, after application, the pattern is at the desired scale. Geometric rules, either universal or specific, may be applied to the pattern to be printed on the transfer surface so that the pattern has the desired form after transfer onto the area of the keratin materials to be treated. The use of such rectification rules is particularly advantageous with a support that has a transfer surface bearing reliefs, in particular in order to match an imprint. Use may be made in particular of specific geometric rules adapted to the area to be treated and/or to the desired pattern.

The invention makes it possible to obtain printed cosmetic compositions suitable for transfer makeup application by simple contact, without addition of solvent, whether the user seeks to transfer just after printing or after a longer or shorter period of storage of the cosmetic composition.

The invention makes it possible to take advantage, for cosmetic compositions, of the performances of laser printing technology, especially in terms of contrast and precision.

### Cosmetic treatment process

Another subject of the invention, according to another of its aspects, in combination with the foregoing, is a process for cosmetically treating, in particular for making up, human keratin materials, in particular the skin, lips, nails, eyelashes, eyebrows or hair, comprising the following steps:
- producing, with the aid of the process as defined above, a cosmetic article,
- applying, by transfer, the composition of the cosmetic article, by applying the support coated with composition to said keratin materials.

Another subject of the invention, according to another of its aspects, in combination with the foregoing, is a process for cosmetically treating, in particular for making up, human keratin materials, in particular the skin, lips, nails, eyelashes, eyebrows or hair, comprising the following steps:
- producing, with the aid of the process as defined above, a cosmetic article,
- removing, in particular with the aid of an applicator, for example a flocked tip, a sponge or a foam, a portion of the cosmetic article, and applying it to the keratin materials.

After transfer of the toner to the keratin materials, the latter may be covered, with the toner, by a protective coating, in particular deposited by spraying. It may be a colorless resin.

When the application of the cosmetic composition takes place by transfer, the invention may enable the user to retouch the pattern once transferred, for example in order to soften the contours thereof, and to smooth the demarcations with the non-madeup area.

### Packaging and application device

Another subject of the invention, according to another of its aspects, in combination with the foregoing, is a device for packaging and applying a cosmetic composition, comprising a support coated with printing obtained by a process as defined above. The pattern present on the support may be polychromatic and correspond for example to a gradation and/or be as defined above.

### Figures

The invention may be better understood from reading the following detailed description of non-limiting exemplary embodiments thereof and from examining the appended drawing, in which:
- figure 1 schematically illustrates an electrophotographic printing process that may be carried out within a laser printer, and
- figure 2 represents an example of a support coated by printing with a cosmetic composition, in accordance with the process according to the invention.

An example of a laser printer that can be used in the process according to the invention is disclosed in application US 2006/0093943 A1.

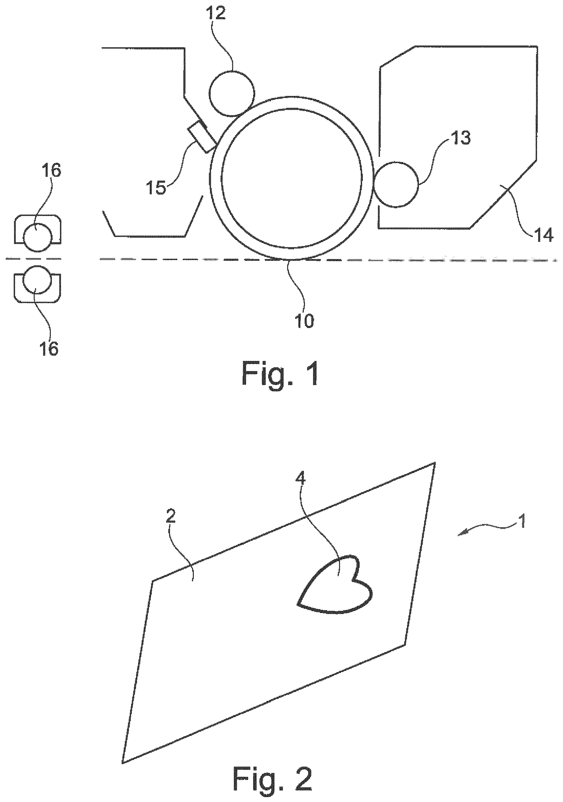

As is known, a laser printer that uses an electrophotographic printing process comprises, as illustrated in figure 1, a drum 10 coated with a photoreceptor which is used to receive an electrostatic latent image, an element 12 for electrically charging the photoreceptor before the exposure thereof to the laser, an element 13 for developing the electrostatic latent image with a toner removed from a cartridge 14 and a cleaning element 15 for cleaning the drum after transfer of the developed image.

In figure 1, the following have not been represented: the laser imager that forms the electrostatic latent image on the drum or the mechanism which transfers the developed image onto an intermediate transfer element, for example a strip circulating in closed loop form, before contact of this intermediate element with a support such as a sheet of paper.

The electrostatic image is, as is widely known, created as a function of the areas where the laser has not irradiated the photoreceptor. The toner particles are selectively deposited on the drum, according to the distribution of the electrostatic charges, in order to reproduce the image to be obtained.

In one variant, the image is formed by magnetophotography, the photoreceptor and the toner being magnetic. Publications describe this process, for example application EP 2 090 935 A1 in paragraphs [0009] to [0014].

Preferably, the laser printer used operates electrophotographically, rather than magnetophotographically. Preferably also, the laser printer is a polychrome printer, rather than a monochrome printer.

A conventional laser printer also comprises a fuser or heating unit 16 comprising a heat roller and a means for controlling the temperature of this heat roller.

Within a printer for the implementation of the process according to the invention, the temperature of the fuser is preferably controlled, either by a hardware intervention, or by software means, in order to heat the printed support to a predetermined operating temperature, for example 60°C, 80°C or 120°C.

The printing support may be of any suitable type and may in particular consist of a sheet material such as a paper or a plastic film.

Represented in figure 2 is a support 2 in the form of sheet material, on one face of which a pattern 4 has been printed electrophotographically using a laser printer according to the process in accordance with the invention.

The support may be, where appropriate, deformable and flexible so as to more readily match the relief of the keratin materials onto which the pattern must be transferred.

The support may be impermeable to water, in order to facilitate the transfer of the pattern after wetting of the surface onto which the pattern must be transferred.

The cosmetic toner(s) deposited by printing may be transferred by bringing them into contact with the area to be made up and pressing thereon.

The transfer may be carried out dry, without prior wetting of the area to be made up or of the printed pattern. As a variant, a transfer compound is applied to at least one of the pattern and of the area to be made up, for example an adhesive, with the view to facilitating the transfer and improving the hold of the composition transferred to the madeup area.

In one exemplary embodiment, the printing is performed directly onto a non-flat transfer surface of the support, corresponding for example to the outer surface of a roller. This roller is for example applied against the intermediate transfer element of the printer, and turns in contact therewith in order to receive the pattern. Next, the roller is moved onto the skin for the transfer.

In another embodiment, the cosmetic composition deposited by printing on the support is removed, for example with an applicator, such as a sponge, a brush, or other, or else with the finger, then applied to the area of the human keratin materials to be made up.

The pattern formed on the surface of the support may be of any type.

The process may comprise a step of choosing and/or making a pattern by a user and of transmitting, by means of a machine connected to the printer that performs the printing, information relating to this pattern.

The machine may be a computer, an advanced portable telephone, also known as a "smartphone", or a tablet computer. The machine may be connected physically and/or by means of a data exchange network to said printer.

The toner or toners are deposited in the form of raster dots and/or of raster lines, so as to form a halftone image, for example a monochromatic or polychromatic image.

The pattern may reproduce the appearance of relief and/or color heterogeneities of the skin, for example freckles or a mole.

The pattern may be colored when observed under white light in the visible region (400 nm - 800 nm). As a variant, the pattern is colorless under white light in the visible region, but appears colored when submitted to a chemical and/or energy stimulus, such as exposure to UV (365 nm - 400 nm).

The printing may use only toners that correspond to primary colors. As a variant, the printing uses both toners of primary colors and at least one toner of non-primary color.

The printing may be monochromatic, but preferably it is carried out as three-color printing or as four-color printing.

The pattern obtained by printing may comprise several areas of different colors, for example according to a gradation. As a variant, the pattern obtained by printing is a flat tint.

### Tests

An HP LaserJet Pro 400 M451 NW printer is used.

A cartridge of an HP LaserJet Pro Color M451NW printer is taken. After opening, the powder present in the cartridge is removed in order to replace it with the "yellow" cosmetic toner composed of the following mixture:

| | |
|---|---|
| - Yellow iron oxide (CI 77492) | 40% |
| - Brilliant yellow FCF aluminum lake on alumina (42/58) (CI 15985 :1 + CI 77002) | 50% |
| - Titanium oxide | 5% |
| - Amorphous silica RN: 7631-86-9: | 5% |

A "blue" cosmetic toner is also produced, composed of the following mixture, that is placed in another cartridge of the printer:

| | |
|---|---|
| - Ultramarine blue (CI 77007) | 90% |
| - Calcium carbonate | 5% |
| - Amorphous silica RN: 7631-86-9: | 5% |

A "red" cosmetic toner is also produced, composed of the following mixture, that is placed in another cartridge of the printer:

| | |
|---|---|
| - Cochineal carmine aluminum lake (CI 75470) | 90% |
| - Calcium carbonate | 5% |
| - Amorphous silica RN: 7631-86-9: | 5% |

The percentages indicated are understood to mean by weight relative to the total weight of the composition forming the cosmetic toner. The toners have, in this example, no fusible material.

The toners are not disinfected and contain no antibacterial agent.

The printing is produced on a support formed by a sheet of transparent type for a laser printer using the process according to the invention, by heating the printed support to an operating temperature of 60°C by controlling the temperature of the fuser.

The printed pattern is applied by transfer onto the skin just after printing. The sheet is placed on the skin with a pressure of 50 g per cm² for 5 seconds. The sheet is then removed.

To complete the treatment, a composition containing a resin is sprayed on, at a distance of 30 cm. To do this, an Elnett brand hair lacquer is used. It is left at rest for 1 minute. The treatment is then complete.

In another example, a multicolor palette is produced on a computer. Then, the printer is used to carry out a deposition of powders according to the process in accordance with the invention. A support covered with various powders is obtained which can be used to make oneself up. In order to do this, a small brush is used to take powder having the desired color.

## Claims

1. A process for producing a cosmetic article by printing using a laser printer comprising a heating unit and at least one cosmetic toner, the process comprising the steps consisting in:
a) producing, with the printer, a printing on a support with the aid of the cosmetic toner,
b) heating the support coated with the printing with the aid of the heating unit to an operating temperature greater than or equal to 60°C, the cosmetic toner comprising a weight content of material that is fusible at the operating temperature of less than 10% relative to the total weight of the toner.

2. The process as claimed in claim 1, wherein the support coated with the printing is heated to an operating temperature greater than or equal to 80°C, preferably greater than or equal to 120°C.

3. The process as claimed in claim 1 or 2, wherein the heating step b) takes place at the same time as the printing step a) or after the printing step a).

4. The process as claimed in any one of the preceding claims, the heating unit being chosen from the group formed by an element in the form of a toner fuser roller, a UV, visible or IR lamp, the emission spectrum being chosen in order to be absorbed by the toner or the support itself, and a combination thereof, the heating unit preferably comprising a toner fuser roller, made of aluminum, coated with a nonstick compound and heated from the inside by a halogen lamp.

5. The process as claimed in any one of the preceding claims, the heating unit having a working temperature greater than or equal to 180°C, better still greater than or equal to 200°C.

6. The process as claimed in either one of claims 3 and 4, the heating unit comprising a toner fuser roller capable of occupying a position spaced apart from the support, in particular by 1 to 20 mm, the process comprising the step consisting in placing the toner fuser roller in the spaced-apart position before the printing step a).

7. The process as claimed in any one of the preceding claims, the cosmetic toner comprising a weight content of material that is fusible at the operating temperature of less than 5% relative to the total weight of the toner.

8. The process as claimed in any one of the preceding claims, the cosmetic toner not comprising any material that is fusible at the operating temperature.

9. The process as claimed in any one of claims 1 to 7, the fusible material being chosen from waxes and fusible polymers or polymers that can be softened at a temperature below 120°C and mixtures thereof.

10. The process as claimed in any one of the preceding claims, the cosmetic toner comprising only compounds that have a melting temperature and/or glass transition temperature Tg above 120°C, included in particular in the group formed by polycarbonates, polyvinylcaprolactams, polyetheretherketones, polyetherketones, polyaryletherketones, polyterephthalates, polysulfones and polyetherimides.

11. The process as claimed in any one of the preceding claims, the cosmetic toner comprising at least one compound chosen from the group formed by at least one colorant, opacifying particles, anticaking particles, transparent particles, scattering white particles, and a mixture thereof.

12. The process as claimed in any one of the preceding claims, the toner having no antibacterial agent.

13. The process as claimed in any one of the preceding claims, the laser printer comprising a plurality of different cosmetic toners, the process consisting in carrying out steps a) and b) several times with the various toners, so as to produce several different printings on the support.

14. A process for cosmetically treating, in particular for making up, human keratin materials, in particular the skin, comprising the following steps:
- producing, with the aid of the process as claimed in any one of claims 1 to 13, a cosmetic article,
- applying, by transfer, the composition of the cosmetic article, by applying the support coated with composition to said keratin materials.

15. A process for cosmetically treating, in particular for making up, human keratin materials, in particular the skin, comprising the following steps:
- producing, with the aid of the process as claimed in any one of claims 1 to 13, a cosmetic article,
- removing, in particular with the aid of an applicator, a portion of the cosmetic article, and
- applying it to the keratin materials.

## Patentansprüche

1. Verfahren zur Herstellung eines kosmetischen Artikels durch Drucken unter Verwendung eines Laserdruckers, der eine Heizeinheit und mindestens einen kosmetischen Toner umfasst, wobei das Verfahren die Schritte umfasst, die darin bestehen:
a) mit dem Drucker einen Druck auf einem Träger mit Hilfe des kosmetischen Toners herzustellen,
b) Erhitzen des mit dem Druck beschichteten Trägers mit Hilfe der Heizeinheit auf eine Betriebstemperatur von mindestens 60 °C, wobei der kosmetische Toner einen Gewichtsanteil an bei der Betriebstemperatur schmelzbarem Material von weniger als 10 %, bezogen auf das Gesamtgewicht des Toners, aufweist.

2. Verfahren nach Anspruch 1, wobei der mit dem Druck beschichtete Träger auf eine Betriebstemperatur von mindestens 80 °C, vorzugsweise von mindestens 120 °C, erhitzt wird.

3. Verfahren nach Anspruch 1 oder 2, wobei der Erwärmungsschritt b) zur gleichen Zeit wie der Druckschritt a) oder nach dem Druckschritt a) erfolgt.

4. Verfahren nach einem der vorhergehenden Ansprüche, wobei die Heizeinheit aus der Gruppe ausgewählt wird, die aus einem Element in Form einer Tonerfixierwalze, einer UV-, sichtbaren oder IR-Lampe, wobei das Emissionsspektrum so gewählt wird, dass es vom Toner oder dem Träger selbst absorbiert wird, und einer Kombination davon besteht, wobei die Heizeinheit vorzugsweise eine Tonerfixierwalze aus Aluminium umfasst, die mit einer Antihaftverbindung beschichtet ist und von innen durch eine Halogenlampe beheizt wird.

5. Verfahren nach einem der vorhergehenden Ansprüche, wobei die Heizeinheit eine Arbeitstemperatur von mindestens 180°C, besser noch von mindestens 200°C aufweist.

6. Verfahren nach einem der Ansprüche 3 und 4, wobei die Heizeinheit eine Tonerfixierwalze umfasst, die eine vom Träger beabstandete Position einnehmen kann, insbesondere um 1 bis 20 mm, wobei das Verfahren den Schritt umfasst, der darin besteht, die Tonerfixierwalze vor dem Druckschritt a) in die beabstandete Position zu bringen.

7. Verfahren nach einem der vorhergehenden Ansprüche, wobei der kosmetische Toner einen Gewichtsanteil an Material, das bei der Betriebstemperatur schmelzbar ist, von weniger als 5 %, bezogen auf das Gesamtgewicht des Toners, aufweist.

8. Verfahren nach einem der vorhergehenden Ansprüche, wobei der kosmetische Toner kein Material enthält, das bei der Betriebstemperatur schmelzbar ist.

9. Verfahren nach einem der Ansprüche 1 bis 7, wobei das schmelzbare Material aus Wachsen und schmelzbaren Polymeren oder Polymeren, die bei einer Temperatur unter 120 °C erweicht werden können, und Mischungen davon ausgewählt wird.

10. Verfahren nach einem der vorhergehenden Ansprüche, wobei der kosmetische Toner nur Verbindungen enthält, die eine Schmelztemperatur und/oder Glasübergangstemperatur Tg über 120°C aufweisen, insbesondere aus der Gruppe der Polycarbonate, Polyvinylcaprolactame, Polyetheretherketone, Polyetherketone, Polyaryletherketone, Polyterephthalate, Polysulfone und Polyetherimide.

11. Verfahren nach einem der vorhergehenden Ansprüche, wobei der kosmetische Toner mindestens eine Verbindung enthält, die aus der Gruppe ausgewählt ist, die aus mindestens einem Farbstoff, Trübungspartikeln, Antiklumppartikeln, transparenten Partikeln, Weißstreupartikeln und einer Mischung davon besteht.

12. Verfahren nach einem der vorhergehenden Ansprüche, wobei der Toner kein antibakterielles Mittel enthält.

13. Verfahren nach einem der vorhergehenden Ansprüche, wobei der Laserdrucker eine Vielzahl verschiedener kosmetischer Toner enthält, wobei das Verfahren darin besteht, die Schritte a) und b) mehrmals mit den verschiedenen Tonern durchzuführen, um mehrere verschiedene Drucke auf dem Träger zu erzeugen.

14. Verfahren zur kosmetischen Behandlung, insbesondere zum Schminken, von menschlichen Keratinmaterialien, insbesondere der Haut, mit den folgenden Schritten:
- Herstellung eines kosmetischen Artikels mit Hilfe des Verfahrens nach einem der Ansprüche 1 bis 13,
- Auftragen der Zusammensetzung des kosmetischen Artikels durch Übertragung, indem der mit der Zusammensetzung beschichtete Träger auf die Keratinmaterialien aufgebracht wird.

15. Verfahren zur kosmetischen Behandlung, insbesondere zum Schminken, von menschlichen Keratinmaterialien, insbesondere der Haut, umfassend die folgenden Schritte:
- Herstellung eines kosmetischen Artikels mit Hilfe des Verfahrens nach einem der Ansprüche 1 bis 13,
- Entnahme, insbesondere mit Hilfe eines Applikators, eines Teils des kosmetischen Artikels, und
- Auftragen auf die Keratinmaterialien.

## Revendications

1. Procédé de production d'un article cosmétique par impression à l'aide d'une imprimante laser comportant une unité de chauffage et au moins un toner cosmétique, le procédé comportant les étapes consistant à :
a) réaliser, avec l'imprimante, une impression sur un support à l'aide du toner cosmétique,
b) chauffer le support revêtu de l'impression à l'aide de l'unité de chauffage à une température d'opération supérieure ou égale à 60°C, le toner cosmétique comportant une teneur massique en matériau fusible à la température d'opération inférieure à 10% par rapport à la masse totale du toner.

2. Procédé selon la revendication 1, dans lequel le support revêtu de l'impression est chauffé à une température d'opération supérieure ou égale à 80°C, de préférence supérieure ou égale à 120°C.

3. Procédé selon l'une quelconque des revendications 1 ou 2, dans lequel l'étape b) de chauffage a lieu simultanément à l'étape a) ou après l'étape a) d'impression.

4. Procédé selon l'une quelconque des revendications précédentes, l'unité de chauffage étant choisie dans le groupe constitué par un élément, sous forme de rouleau de cuisson du toner, une lampe UV, visible ou IR, le spectre d'émission étant choisi pour être absorbé par le toner ou le support lui-même, et une combinaison de ceux-ci, l'unité de chauffage comportant de préférence, un rouleau de cuisson du toner, réalisé en aluminium, revêtu d'un composé antiadhésif et chauffé de l'intérieur par une lampe halogène.

5. Procédé selon l'une quelconque des revendications précédentes, l'unité de chauffage ayant une température de fonctionnement supérieure ou égale à 180°C, mieux supérieure ou égale à 200°C.

6. Procédé selon l'une quelconque des revendications 3 et 4, l'unité de chauffage comportant un rouleau de cuisson du toner apte à occuper une position écartée du support, notamment de 1 à 20 mm, le procédé comportant l'étape consistant à disposer le rouleau de cuisson du toner dans la position écartée avant l'étape a) d'impression.

7. Procédé selon l'une quelconque des revendications précédentes, le toner cosmétique comportant une teneur massique en matériau fusible à la température d'opération inférieure à 5% par rapport à la masse totale du toner.

8. Procédé selon l'une quelconque des revendications précédentes, le toner cosmétique ne comportant pas de matériau fusible à la température d'opération.

9. Procédé selon l'une quelconque des revendications 1 à 7, le matériau fusible étant choisi parmi les cires et les polymères fusibles ou pouvant être ramollis à une température inférieure à 120°C et leurs mélanges.

10. Procédé selon l'une quelconque des revendications précédentes, le toner cosmétique comportant uniquement des composés présentant une température de fusion et/ou température de transition vitreuse Tg supérieure à 120°C, compris notamment dans le groupe constitué par les polycarbonates, les polyvinylcaprolactame, les polyétherethercétone, les polyéthercétone, les polyaryléthercétone, les polytéréphtalate, les polysulfone et les polyétherimide.

11. Procédé selon l'une quelconque des revendications précédentes, le toner cosmétique comportant au moins un composé choisi dans le groupe constitué par au moins une matière colorante, des particules opacifiantes, des particules anti-agglomérantes, des particules transparentes, des particules blanches diffusantes, et un mélange de celles-ci.

12. Procédé selon l'une quelconque des revendications précédentes, le toner étant dépourvu d'agent anti-bactérien.

13. Procédé selon l'une quelconque des revendications précédentes, l'imprimante laser comportant une pluralité de toners cosmétiques différents, le procédé consistant à mettre en œuvre plusieurs fois les étapes a) et b) avec les différents toners, de manière à réaliser plusieurs impressions différentes sur le support.

14. Procédé de traitement cosmétique, notamment de maquillage, de matières kératiniques humaines, notamment la peau, comportant les étapes suivantes :
- réaliser, à l'aide du procédé selon l'une quelconque des revendications 1 à 13, un article cosmétique,
- appliquer par transfert la composition de l'article cosmétique, en appliquant le support revêtu de composition sur lesdites matières kératiniques.

15. Procédé de traitement cosmétique, notamment de maquillage, de matières kératiniques humaines, notamment la peau, comportant les étapes suivantes :
- réaliser, à l'aide du procédé selon l'une quelconque des revendications 1 à 13, un article cosmétique,
- prélever, notamment à l'aide d'un applicateur, une portion de l'article cosmétique, et
- l'appliquer sur les matières kératiniques.
